(19) Europäisches Patentamt
European Patent Office
Office européen des brevets

(11) Veröffentlichungsnummer: **0 019 730**
**B1**

(12) # EUROPÄISCHE PATENTSCHRIFT

(45) Veröffentlichungstag der Patentschrift: **12.05.82**

(21) Anmeldenummer: **80102288.0**

(22) Anmeldetag: **28.04.80**

(51) Int. Cl.³: **A 61 K 31/415,** A 01 N 43/50 // C07D233/60, C07C49/16, C07C49/167, C07C49/175, C07C143/68

(54) **Antimykotisches Mittel, Verfahren zur Herstellung dieses Mittels.**

(30) Priorität: **10.05.79 DE 2918897**

(43) Veröffentlichungstag der Anmeldung: **10.12.80 Patentblatt 80/25**

(45) Bekanntmachung des Hinweises auf die Patenterteilung: **12.05.82 Patentblatt 82/19**

(84) Benannte Vertragsstaaten: **AT BE CH DE FR GB IT LI NL SE**

(56) Entgegenhaltungen:
**EP-A-0 009 707**
**DE-A-2 632 601**
**DE-A-2 632 602**
**DE-A-2 632 603**
**DE-A-2 811 916**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal-1 (DE)**
Erfinder: **Büchel, Karl Heinz, Dr., Bergerheide 62, D-5600 Wuppertal-1 (DE)**
Erfinder: **Stetter, Jörg, Dr., Gellertweg 4, D-5600 Wuppertal-1 (DE)**
Erfinder: **Plempel, Manfred, Dr., Pahlkestrasse 5, D-5600 Wuppertal-1 (DE)**
Erfinder: **Haller, Ingo, Dr., Viktoriastrasse 99, D-5600 Wuppertal-1 (DE)**

nmerkung: Innerhalb von neun Monaten nach der Bekanntmachung des Hinweises auf die Erteilung des europäischen atents im Europäischen Patentblatt kann jedermann beim Europäischen Patentamt gegen das erteilte europäische Patent inspruch einlegen. Der Einspruch ist schriftlich einzureichen und zu begründen. Er gilt erst als eingelegt, wenn die inspruchsgebühr entrichtet worden ist (Art. 99(1) Europäisches Patentübereinkommen).

BUNDESDRUCKEREI BERLIN

Antimykotisches Mittel und Verfahren zur Herstellung dieses Mittels

Die Erfindung betrifft antimykotische Mittel, welche neue fluorierte 1-Imidazolyl-butan-Derivate als Wirkstoff enthalten sowie Verfahren zur Herstellung dieser Mittel.

Es ist bereits bekanntgeworden, daß chlorierte und bromierte 1-Imidazolyl-butan-Derivate gute antimykotische Eigenschaften aufweisen (vgl. DE-A-2 632 601). Die Wirksamkeit dieser Verbindungen ist jedoch, insbesondere in-vivo gegen Candida, nicht immer ganz befriedigend.

Es wurde gefunden, daß die neuen fluorierten 1-Imidazolyl-butan-Derivate der allgemeinen Formel I

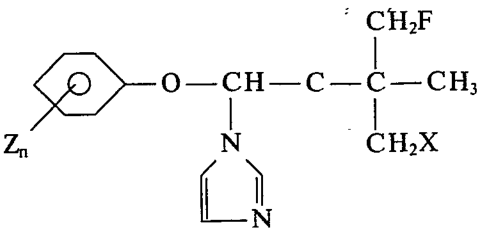

(I)

in welcher

B für die Ketogruppe oder die CH(OH)-Gruppierung steht,
X für Wasserstoff oder Fluor steht,
Z für Halogen, Alkyl mit 1–4 C-Atomen, Nitro, Cyano, Alkoxycarbonyl mit 1–4 C-Atomen im Alkylteil sowie für gegebenenfalls durch Halogen substituiertes Phenyl steht und
n für 0, 1, 2 oder 3 steht,

und deren physiologisch verträglichen Säureadditions-Salze und Metallsalz-Komplexe gute antimikrobielle, insbesondere antimykotische Eigenschaften aufweisen.

Diejenigen Verbindungen der allgemeinen Formel I, in welchen B für die CH(OH)-Gruppierung steht, besitzen zwei asymmetrische Kohlenstoffatome; sie können deshalb in den beiden geometrischen Isomeren (threo- und erythro-Form) vorliegen, die in unterschiedlichen Mengenverhältnissen anfallen können. In beiden Fällen liegen sie als optische Isomeren vor. Sämtliche Isomeren werden erfindungsgemäß beansprucht.

Überraschenderweise zeigen die erfindungsgemäß verwendbaren fluorierten 1-Imidazolyl-butan-Derivate der allgemeinen Formel I eine bessere antimykotische Wirksamkeit, insbesondere in vivo gegen Candida, als die bekannten chlorierten und bromierten 1-Imidazolyl-butan-Derivate, die chemisch die naheliegendsten Verbindungen sind. Die erfindungsgemäß verwendbaren Stoffe stellen somit eine Bereicherung der Pharmazie dar.

Bevorzugt sind diejenigen fluorierten 1-Imidazolyl-butan-Derivate, in denen Z für Fluor, Chlor, Brom, Jod, Methyl, Ethyl, Nitro, Cyano, Methoxycarbonyl, Ethoxycarbonyl, Phenyl oder Chlorphenyl steht, der Index n für 0, 1 oder 2 steht und B sowie X die in der Erfindungsdefinition angegebene Bedeutung haben.

Im einzelnen seien außer den bei den Herstellungsbeispielen genannten Verbindungen die folgenden Verbindungen der allgemeinen Formel I genannt:

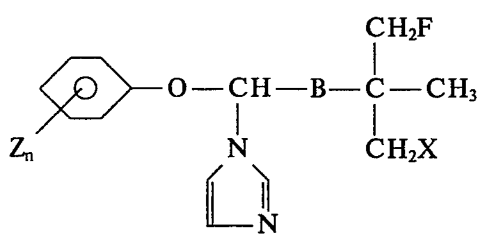

(I)

| $Z_n$ | X | B |
|---|---|---|
| — | H | CO |
| 2-F | H | CO |
| 3-F | H | CO |
| 4-F | H | CO |
| 2-Cl | H | CO |
| 3-Cl | H | CO |
| 2-Br | H | CO |
| 3-Br | H | CO |
| 2-$CH_3$ | H | CO |
| 4-$CH_3$ | H | CO |
| 2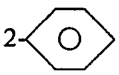 | H | CO |
| 4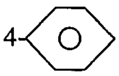 | H | CO |
| 4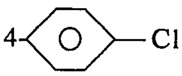 | H | CO |
| 2-$NO_2$ | H | CO |
| 4-CN | H | CO |
| 4-$COOCH_3$ | H | CO |
| 4-$COOC_2H_5$ | H | CO |
| 4-J | H | CO |
| 4-Cl, 2-$CH_3$ | H | CO |
| 4-$CH_3$, 2-Cl | H | CO |
| — | H | CH(OH) |
| 2-F | H | CH(OH) |
| 3-F | H | CH(OH) |
| 4-F | H | CH(OH) |
| 2-Cl | H | CH(OH) |
| 3-Cl | H | CH(OH) |
| 2-Br | H | CH(OH) |
| 3-Br | H | CH(OH) |
| 2-$CH_3$ | H | CH(OH) |
| 4-$CH_3$ | H | CH(OH) |
| 2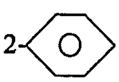 | H | CH(OH) |
| 4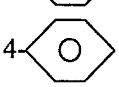 | H | CH(OH) |
| 4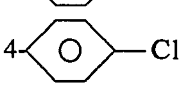 | H | CH(OH) |
| 2-$NO_2$ | H | CH(OH) |
| 4-CN | H | CH(OH) |
| 4-$COOCH_3$ | H | CH(OH) |
| 4-$COOC_2H_5$ | H | CH(OH) |
| 4-J | H | CH(OH) |
| 4-Cl, 2-$CH_3$ | H | CH(OH) |
| 4-$CH_3$, 2-Cl | H | CH(OH) |
| — | F | CO |
| 2-F | F | CO |
| 3-F | F | CO |
| 3-Cl | F | CO |
| 2-Br | F | CO |
| 3-Br | F | CO |
| 4-Br | F | CO |
| 2-$CH_3$ | F | CO |
| 4-$CH_3$ | F | CO |
| 2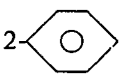 | F | CO |
| 4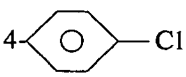 | F | CO |
| 2-$NO_2$ | F | CO |
| 4-$NO_2$ | F | CO |
| 4-CN | F | CO |
| 4-$COOCH_3$ | F | CO |
| 4-$COOC_2H_5$ | F | CO |
| 4-J | F | CO |
| 4-Cl, 2-$CH_3$ | F | CO |
| — | F | CH(OH) |
| 2-F | F | CH(OH) |
| 3-F | F | CH(OH) |
| 3-Cl | F | CH(OH) |
| 2-Br | F | CH(OH) |
| 3-Br | F | CH(OH) |
| 4-Br | F | CH(OH) |
| 2-$CH_3$ | F | CH(OH) |
| 4-$CH_3$ | F | CH(OH) |
| 2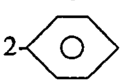 | F | CH(OH) |
| 4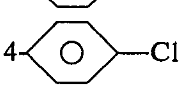 | F | CH(OH) |
| 2-$NO_2$ | F | CH(OH) |

Fortsetzung

| $Z_n$ | X | B |
|---|---|---|
| 4-$NO_2$ | F | CH(OH) |
| 4-CN | F | CH(OH) |
| 4-$COOCH_3$ | F | CH(OH) |
| 4-$COOC_2H_5$ | F | CH(OH) |
| 4-J | F | CH(OH) |
| 4-Cl, 2-$CH_3$ | F | CH(OH) |
| 4-$CH_3$, 2-Cl | F | CH(OH) |
| 4-F | F | CH(OH) |

Die erfindungsgemäß zu verwendenden Wirkstoffe, deren Säureadditions-Salze und Metallsalz-Komplexe sind nocht nicht bekannt. Sie können jedoch gemäß einem eigenen Vorschlag hergestellt werden, indem man Halogenetherketone der allgemeinen Formel II

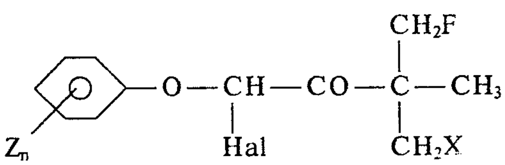

(II)

in welcher

X, Z und n die oben angegebene Bedeutung haben und

Hal für Halogen, vorzugsweise Chlor oder Brom steht,

mit Imidazol in Gegenwart eines Säurebindemittels, wie beispielsweise Natriumcarbonat oder ein Überschuß an Imidazol, und gegebenenfalls in Gegenwart eines inerten organischen Lösungsmittels, wie beispielsweise Aceton oder Acetonitril, bei Temperaturen zwischen 60 und 120°C umsetzt und gegebenenfalls noch die erhaltenen Keto-Derivate nach bekannten Methoden reduziert, wie z. B. durch Umsetzung mit komplexen Hydriden, wie insbesondere Natriumborhydrid, gegebenenfalls in Gegenwart eines polaren organischen Lösungsmittels, wie beispielsweise Alkohole, bei Temperaturen zwischen 0 und 30°C; oder durch Umsetzung mit Aluminiumisopropylat in Gegenwart eines inerten organischen Lösungsmittels, wie beispielsweise Isopropanol, bei Temperaturen zwischen 20 und 120°C. Die Aufarbeitung erfolgt in üblicher Weise; gegebenenfalls wird das Salz oder ein Metallkomplex hergestellt. In manchen Fällen erweist es sich als vorteilhaft, die Verbindungen der allgemeinen Formel I über ihre Salze in reiner Form zu erhalten.

Die Halogenetherketone der allgemeinen Formel II sind noch nicht bekannt. Sie können jedoch nach bekannten Verfahren (vgl. z. B. DE-A-2 632 603) erhalten werden, indem man z. B. bekannte Phenole der allgemeinen Formel III

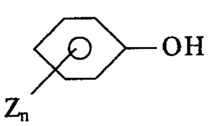

(III)

in welcher

Z und n die oben angegebene Bedeutung haben,

mit einem Halogenketon der allgemeinen Formel IV

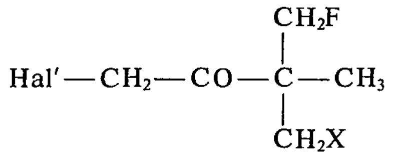

(IV)

in welcher

X die oben angegebene Bedeutung hat und

Hal' für Chlor oder Brom steht,

umsetzt. Das noch verbliebene aktive Wasserstoffatom wird anschließend in üblicher Weise gegen Halogen ausgetauscht (vgl. auch die Herstellungsbeispiele).

Die Halogenketone der allgemeinen Formel IV sind ebenfalls noch nicht bekannt. Sie können jedoch auf allgemein übliche und bekannte Weise erhalten werden, indem man Fluorderivate des 3,3-Dimethyl-butan-2-ons der allgemeinen Formel V

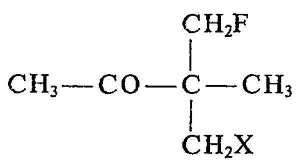

(V)

in welcher

X die oben angegebene Bedeutung hat,

mit Chlor oder Brom in Gegenwart eines inerten organischen Lösungsmittels, wie beispielsweise Ether oder chlorierte Kohlenwasserstoffe, bei Raumtemperatur versetzt (vgl. auch die Herstellungsbeispiele), oder mit üblichen Chlorierungsmitteln, wie beispielsweise Sulfurylchlorid bei 20 bis 60° C umsetzt.

Die Fluorderivate des 3,3-Dimethyl-butan-2-ons der allgemeinen Formel V sind aus der DE-A-2 843 767 bekannt. Man erhält sie, wenn man Sulfonsäureester der allgemeinen Formel VI

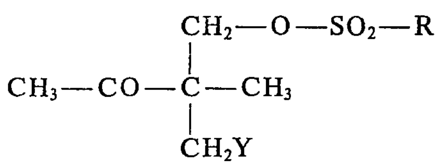

(VI)

in welcher

R für Alkyl mit 1 bis 4 Kohlenstoffatomen, insbesondere Methyl, oder Aryl mit 6 bis 12 Kohlenstoffatomen, insbesondere Phenyl oder Tolyl, steht und
Y für Wasserstoff oder die Gruppe $-O-SO_2-R$ steht,

mit Metallfluoriden, wie beispielsweise Natrium- und Kaliumfluorid, in Gegenwart eines polaren organischen Lösungsmittels, wie beispielsweise Di-, Tri- oder Tetraethylenglykol, bei Temperaturen zwischen 80 und 250° C umsetzt (vgl. auch die Herstellungsbeispiele).

Die Sulfonsäureester der allgemeinen Formel VI sind teilweise bekannt (vgl. J. Org. Chem., 35, 2391 [1970]). Die noch nicht beschriebenen Verbindungen können nach literaturbekannten Verfahren aus den entsprechenden Hydroxybutanonen und Sulfochloriden in Gegenwart von Basen hergestellt werden (siehe z. B. Houben—Weyl, Methoden der Org. Chemie, Bd. IX, S. 388 und 663, sowie die Angaben bei den Herstellungsbeispielen).

Zur Herstellung von Säureadditionssalzen der Verbindungen der allgemeinen Formel I kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure sowie Sulfonsäuren, wie z. B. p-Toluolsulfonsäure und 1,5-Naphthalindisulfonsäure.

Die Salze der Verbindungen der allgemeinen Formel I können in einfacher Weise nach üblichen

Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genant seien. Als Anionen der Salze kommen solche in Betracht, die sich von physiologischen Säuren ableiten. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure.

Die Metallsalzkomplexe der Verbindungen der allgemeinen Formel I können in einfacher Weise nach üblichen Verfahren erhalten werden, so z. B. durch Lösen des Metallsalzes in Alkohol, z. B. Äthanol, und Hinzufügen zur Verbindung der allgemeinen Formel I. Man kann Metallsalzkomplexe in bekannter Weise, z. B. durch Abfiltrieren, Isolieren und gegebenenfalls durch Umkristallisieren reinigen.

Die Wirkstoffe der erfindungsgemäßen Mittel der allgemeinen Formel I, ihre Säureadditions-Salze und Metallsalz-Komplexe weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemäße Wirkstoffe enthalten sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen

Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Sacharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, wähernd in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgend.

## Beispiel A

### Antimykotische in-vitro-Wirksamkeit

### Versuchsbeschreibung

Die in-vitro-Prüfung wurde im Reihenverdünnungstest mit Keiminokula von durchschnittlich 5 × 10 Keimen/ml Substrat durchgeführt. Als Nährmedium dienten

a) für Dermatophyten und Schimmelpilze: Sabourand's milieu d'épresive,
b) für Hefen: Isotonic Sensitest-Broth von Oxid.

Die Bebrütungstemperatur betrug 28° C; Bebrütungszeit war 24 Stunden bei Hefen und 96 Stunden bei Dermatophyten und Schimmelpilzen.

In dieser Versuchsanordnung zeigen die erfindungsgemäßen Mittel gute MHK-Werte.

## Beispiel B

### Antimikrobielle in-vivo-Wirksamkeit (oral) bei Mäuse-Candidose

### Versuchsbeschreibung

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50–100 mg/kg Körpergewicht der Präparate oral behandelt.

### Ergebnis

Unbehandelte Tiere starben 3 bis 6 Tage post infectionem. Die Überlebensrate am 6. Tag post infectionem betrug bei unbehandelten Kontrolltieren etwa 5%. Die nächstliegenden bekannten Verbindungen zeigen keine Wirkung oder nur Spurenwirkung, während die erfindungsgemäßen Mittel Wirkung (≥60% Überlebende am 6. Tage p. i.) bis gute Wirkung (≥80% Überlebende am 6. Tage p. i.) zeigen.

## Beispiel 1

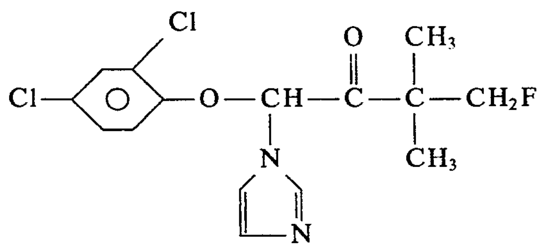

× HCl

157,5 g (0,44 Mol) 1-Brom-1-(2,4-dichlorphenoxy)-3,3-dimethyl-4-fluor-2-butanon werden in 500 ml Acetonitril gelöst und zu einer Lösung von 109 g (1,6 Mol) Imidazol in 600 ml Acetonitril getropft. Man erhitzt 10 Stunden unter Rückfluß. Danach wird das Lösungsmittel im Wasserstrahlvakuum abdestilliert, der Rückstand in 1000 ml Methylenchlorid aufgenommen und dreimal mit je 500 ml Wasser gewaschen. Die organische Phase wird über Natriumsulfat getrocknet, filtriert und im Wasserstrahlvakuum durch Abdestillieren des Lösungsmittels eingeengt. Der Rückstand wird in 500 ml Ethanol gelöst, mit je 40 ml konzentrierter Salzsäure versetzt und das Lösungsmittel im Wasserstrahlvakuum abdestilliert. Der Rückstand wird mit 500 ml Ether verrührt, wobei es zur Kristallisation kommt. Man erhält 66 g (39,3% der Theorie) 1-(2,4-Dichlorphenoxy)-3,3-dimethyl-4-fluor-1-(imidazol-1-yl)-2-butanon-hydrochlorid vom Schmelzpunkt 91–110°C.

### Herstellung der Vorstufe

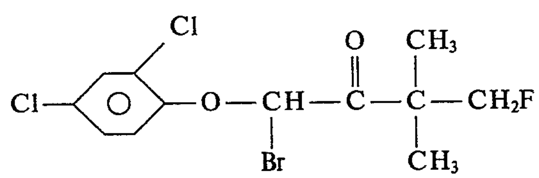

244,6 g (0,88 Mol) rohes 1-(2,4-Dichlorphenoxy)-3,3-dimethyl-4-fluor-2-butanon werden in 700 ml Chloroform gelöst und bei 30°C tropfenweise mit 46 ml Brom so versetzt, daß laufend Entfärbung eintritt. Nach beendeter Zugabe läßt man 30 Minuten nachrühren und engt dann durch Abdestillieren des Chloroforms im Vakuum ein. Man erhält 315 g (100% der Theorie) 1-Brom-1-(2,4-dichlorphenoxy)-3,3-dimethyl-4-fluor-2-butanon als viskoses Öl, das roh weiter umgesetzt werden kann.

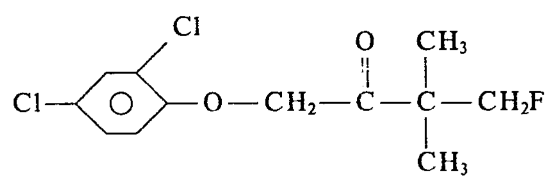

Zu 140 g (1 Mol) Kaliumcarbonat und 163 g (1 Mol) 2,4-Dichlorphenol in 1000 ml Aceton tropft man in der Siedehitze eine Lösung von 197 g (1 Mol) 1-Brom-3,3-dimethyl-4-fluor-2-butanon in 100 ml Aceton. Man läßt 20 Stunden unter Rückfluß rühren, kühlt ab und filtriert. Das Filtrat wird durch Abdestillieren des Acetons im Wasserstrahlvakuum eingeengt. Der Rückstand wird in 500 ml Toluol gelöst, mit 500 ml 10%iger Natronlauge und zweimal mit je 250 ml Wasser gewaschen, über Natriumsulfat getrocknet und durch Abdestillieren des Toluols im Wasserstrahlvakuum eingeengt. Man erhält 244,6 g (88% der Theorie) 1-(2,4-Dichlorphenoxy)-3,3-dimethyl-4-fluor-2-butanon als hochviskoses Öl, das roh weiter umgesetzt werden kann.

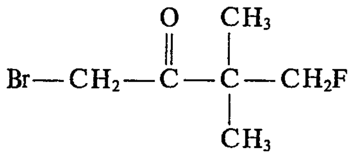

118 g (1 Mol) 3,3-Dimethyl-4-fluor-2-butanon werden in 600 ml Chloroform gelöst und tropfenweise so mit 52 ml Brom versetzt, daß laufend Entfärbung eintritt. Nach beendeter Zugabe läßt man 30 Minuten weiterrühren. Danach wird durch Abdestillieren des Lösungsmittels im Vakuum eingeengt. Man erhält 197 g (100% der Theorie) 1-Brom-3,3-dimethyl-4-fluor-2-butanon vom Siedepunkt 85–90° C/12 mm Hg-Säule, das roh weiter umgesetzt werden kann.

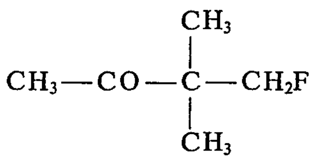

Zu der in einem Dreihals-Rührkolben mit absteigendem Kühler befindlichen Suspension von 23,2 g (0,4 Mol) trockenem Kaliumfluorid in 400 ml dest. Tetraethylenglykol werden bei 160° C und 20 mbar 38,8 g (0,2 Mol) 2,2-Dimethyl-2-oxobutyl-methansulfonat im Verlauf von 2 Stunden zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man erhält 20,9% (89% der Theorie) 3,3-Dimethyl-4-fluor-2-butanon, vom Siedepunkt 130–134° C.

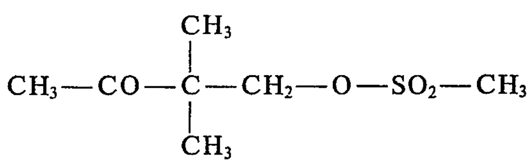

232 g (2 Mol) 3,3-Dimethyl-4-hydroxy-2-butanon (z. Herstellung vgl. Beilstein, H 1 E III 3239, IV, 4030, und Bull. Soc. Chim. France, 1964, 2849) werden in 700 ml absolutem Pyridin bei 0 bis 5° C mit 229 g (2 Mol) Methansulfochlorid umgesetzt. Nach 12 Stunden Stehen bei 20° C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxo-butyl-methansulfonat vom Siedepunkt 106–120° C/0,12 mm Hg-Säule.

Beispiel 2

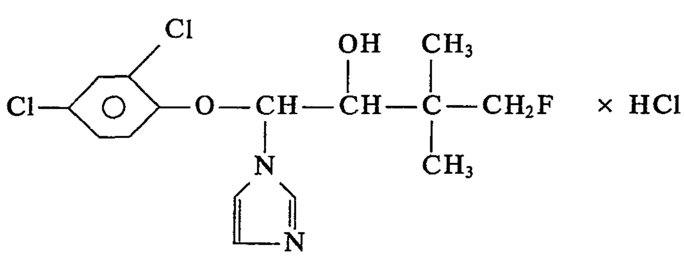

44 g (0,1275 Mol) 1-(2,4-Dichlorphenoxy)-3,3-dimethyl-4-fluor-1-(imidazol-1-yl)-2-butanon (Beispiel ) werden in 300 ml Methanol gelöst und bei 0 bis 5° C mit 6,3 g Natriumborhydrid versetzt. Man läßt 15 tunden bei Raumtemperatur nachrühren, tropft 60 ml konzentrierte Salzsäure unter Eiskühlung zu, errührt 10 Stunden bei Raumtemperatur und destilliert das Lösungsmittel im Wasserstrahlvakuum ab. er Rückstand wird in 250 ml Methylenchlorid aufgenommen, in 500 ml wäßrige, gesättigte

Natriumhydrogencarbonatlösung eingerührt, die Methylenchloridphase abgetrennt, dreimal mit je 100 ml Wasser gewaschen, die organische Phase über Natriumsulfat getrocknet und das Lösungsmittel abdestilliert. Das zurückbleibende Öl wird in 200 ml Ether aufgenommen, 50 ml etherische Salzsäure zugegeben und das Lösungsmittel abdestilliert. Man erhält 28,2 g (58% der Theorie) 1-(2,4-Dichlorphenoxy)-3,3-dimethyl-4-fluor-1-(imidazol-1-yl)-2-butanol-hydrochlorid vom Schmelzpunkt 184–210° C als Diastereomerengemisch.

Beispiel 3

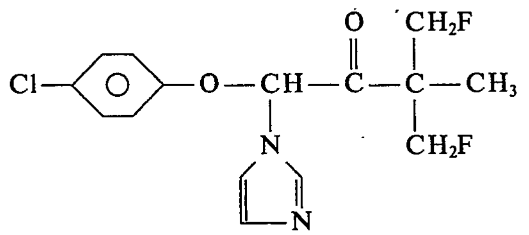

61,5 g (0,18 Mol) 3,3-Bisfluormethyl-1-brom-1-(4-chlorphenoxy)-butan-2-on werden mit 27,2 g (0,4 Mol) Imidazol in 500 ml Acetonitril 4 Stunden bei 45° C gerührt. Das Lösungsmittel wird im Wasserstrahlvakuum abdestilliert, das zurückbleibende Öl in 500 ml Methylenchlorid aufgenommen, die organische Phase zweimal mit 1000 ml Wasser gewaschen, über Natriumsulfat getrocknet und das Lösungsmittel abdestilliert. Das Öl wird in Aceton aufgenommen, mit 36 g (0,1 Mol) 1,5-Naphthalindisulfonsäure-tetrahydrat versetzt und der entstehende Niederschlag abgesaugt. Der Niederschlag (das 1,5-Naphthalindisulfonat des Beispiels 3) wird mit Natriumhydrogencarbonatlösung behandelt. Man erhält 14 g (24% der Theorie) 3,3-Bisfluormethyl-1-(4-chlorphenoxy)-1-(imidazol-1-yl)-butan-2-on als zähflüssiges Öl.

Herstellung der Vorstufen

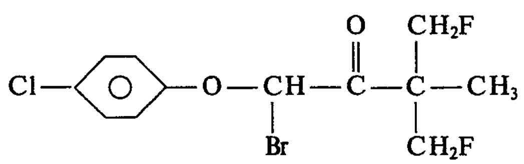

Entsprechend Beispiel 1 durch Umsetzung von 3,3-Bis-fluormethyl-1-(4-chlorphenoxy)-butan-2-on mit Brom.

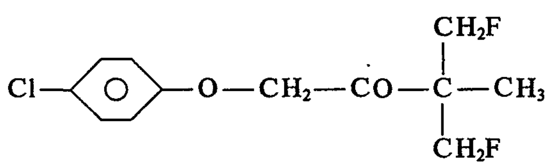

Entsprechend Beispiel 1 durch Umsetzung von 3,3-Bisfluormethyl-butan-2-on mit Brom.

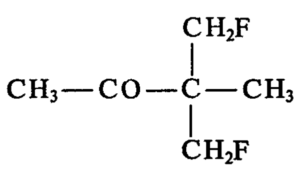

In einem Dreihalskolben mit Rührer, Tropftrichter und Liebigkühler mit gekühlter Vorlage werden 400 ml Tetraethylenglykol und 46,4 g Kaliumfluorid (0,8 Mol) vorgelegt und auf 170° C aufgeheizt. Man legt an den Vorstoß des Liebigkühlers ein Wasserstrahlvakuum (Druck ca. 20 bis 30 mbar) an. Dann werden während 45 Minuten 57,6 g (0,2 Mol) 2-Acetyl-2-methyl-propan-1,3-diol-bismethansulfat, gelöst in 100 ml Tetraethylenglykol, zugetropft. Das entstehende 3,3-Bisfluormethyl-butan-2-on wird während der Reaktion in die gekühlte Vorlage abdestilliert. Nach dem Zutropfen wird noch während 1 Stunde bei 175° C weiter destilliert.

Das aufgefangene Destillat wird anschließend redestilliert. Man erhält 14 g (ca. 51,5% der Theorie) 3,3-Bisfluormethyl-butan-2-on vom Siedepunkt 43–46° C/12 mm Hg-Säule.

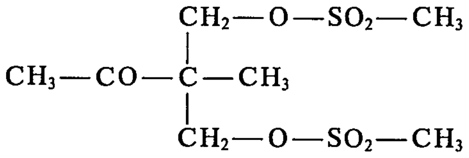

66 g (0,5 Mol) 3-Oxa-2,2-bis-(hydroxymethyl)-butan (zur Herstellung vgl. Beilstein, H 1, E III 3306, IV, 4132, und J. Chem. Soc., London, 1932, 2671) werden in 300 ml 1,2-Dichlorethan gelöst, 114,5 g (1 Mol) Methansulfonsäurechlorid zugetropft und bei 0 bis 5°C 158 g (2 Mol) Pyridin zugetropft. Man läßt 15 Stunden bei Raumtemperatur nachrühren und gibt den Ansatz auf 600 ml Eiswasser und 100 ml konz. Salzsäure. Dabei fällt ein Feststoff aus, der abgesaugt wird. Die wäßrige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Ether suspendiert. Der Rückstand wird abgesaugt und mit 100 ml Ether gewaschen.

Man erhält 100 g (ca. 70% der Theorie) 2-Acetyl-2-methyl-propan-1,3-diol-bis-methansulfonat vom Schmelzpunkt 105 bis 108°C.

In entsprechender Weise werden die nachfolgenden Beispiele der allgemeinen Formel I

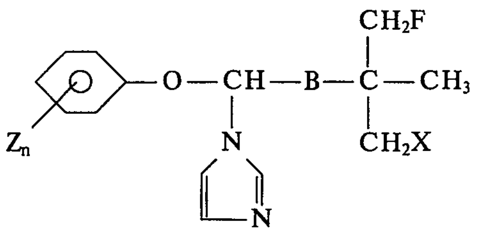

(I)

erhalten:

| Beispiel Nr. | $Z_n$ | B | X | Schmelzpunkt (°C) bzw. Siedepunkt (°C)/mm Hg-Säule |
|---|---|---|---|---|
| 4 | 4-Cl | CO | H | Öl |
| 5 | 4-Cl | CO | H | 260–65 (× 1/2 NDS) |
| 6 | 4-Br | CO | H | 142 (× HCl) |
| 7 | 4 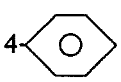 | CO | F | Öl |
| 8 | 4-Cl | CO | F | 250 (× 1/2 NDS) |
| 9 | 4-F | CO | F | 245–50 (× 1/2 NDS) |
| 10 | 4-Cl | CH(OH) | H | 148–52 |
| 11 | 4-Br | CH(OH) | H | 160–62 |
| 12 | 4 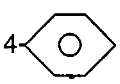 | CH(OH) | F | 151 |
| 13 | 4-Cl | CH(OH) | F | 137 |
| 14 | 2-Cl | CO | F | 206–10 (× 1/2 NDS) |
| 15 | 2,3-$Cl_2$ | CO | F | 198–202 (× 1/2 NDS) |
| 16 | 2-Cl, 4-$CH_3$ | CO | F | 204 (× 1/2 NDS) |
| 17 | 2,4-$CL_2$ | CH(OH) | F | 106–09 (A-Form) |
| 18 | 2-Cl | CH(OH) | F | 110–19 (A-Form) |

NDS = 1,5-Naphthalindisulfonsäure

A-Form = eine der beiden möglichen geometrischen Isomeren

Fortsetzung

| Beispiel Nr. | $Z_n$ | B | X | Schmelzpunkt (°C) bzw. Siedepunkt (°C)/mm Hg-Säule |
|---|---|---|---|---|
| 19 | 4- 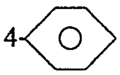 | CO | H | 87–106 |
| 20 | 4- 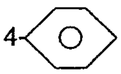 | CH(OH) | H | 120–122 |
| 21 | 4-F | CH(OH) | F | 98–112 |
| 22 | 2,4-$Cl_2$ | CO | F | 124 |
| 23 | 4-Br | CO | F | 260 (× 1/2 NDS) |
| 24 | 4- 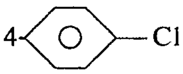 | CO | F | Öl |
| 25 | 3-Cl | CO | F | Öl |
| 26 | 4-Br | CH(OH) | F | 161–63 |
| 27 | 4-Cl, 2-$CH_3$ | CH(OH) | F | 108–15 |
| 28 | 4-$COOC_2H_5$ | CO | F | 243–45 (× 1/2 NDS) |
| 29 | 4- 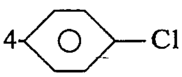 | CH(OH) | F | 151–65 |
| 30 | 3-Cl | CH(OH) | F | 239–43 (× 1/2 NDS) |
| 31 | 4-$CH_3$ | CH(OH) | F | 1[illegible]3–47 |
| 32 | 4-$NO_2$ | CH(OH) | F | 168–73 |
| 33 | 4-$CH_3$ | CO | F | 260 (× 1/2 NDS) |
| 34 | 4-$NO_2$ | CO | F | >260 (× 1/2 NDS) |
| 35 | 4-$COOCH_3$ | CO | F | 250–52 (× 1/2 NDS) |
| 36 | 2-F | CO | H | 135 (× HCl) |
| 37 | — | CO | H | 153 (× HCl) |
| 38 | — | CH(OH) | H | 137 |
| 39 | 2- 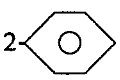 | CO | F | 223–25 (× 1/2 NDS) |
| 40 | 4-CN | CO | F | 256 (× 1/2 NDS) |
| 41 | 2- 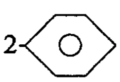 | CH(OH) | F | 97–107 |
| 42 | 4-$COOCH_3$ | CH(OH) | F | 135–47 |
| 43 | 3-Br | CO | F | 245 (× 1/2 NDS) |
| 44 | 4-$COOC_2H_5$ | CH(OH) | F | 123–27 |
| 45 | 4-CN | CH(OH) | F | 107–17 |
| 46 | 3,4-$Cl_2$ | CO | H | 156–60 (Zers.) (× HCl) |

**Patentansprüche**

1. Antimykotisches Mittel, gekennzeichnet durch einen Gehalt von mindestens einem fluorierten 1-Imidazol-yl-butan-Derivat der allgemeinen Formel I

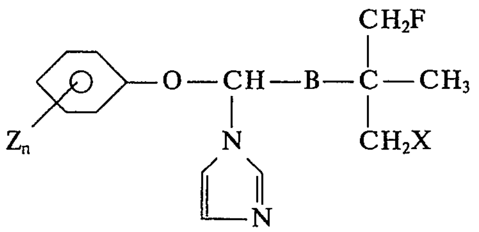

(I)

in welcher

B für die Ketogruppe oder die CH(OH)-Gruppierung steht,
X für Wasserstoff oder Fluor steht,
Z für Halogen, Alkyl mit 1–4 C-Atomen, Nitro, Cyano, Alkoxycarbonyl mit 1–4 C-Atomen im Alkylteil sowie für gegebenenfalls durch Halogen substituiertes Phenyl steht und
n für 0, 1, 2 oder 3 steht,

und/oder deren physiologisch verträglichen Säureadditions-Salzen und/oder Metallkomplexen.

2. Antimykotisches Mittel gemäß Anspruch 1, gekennzeichnet durch einen Gehalt an mindestens einem fluorierten 1-Imidazolylbutan-Derivat der allgemeinen Formel I in Anspruch 1, in welcher

Z für Halogen, Methyl, Ethyl, Nitro, Cyano, Methoxycarbonyl, Ethoxycarbonyl, Phenyl oder Chlorphenyl steht,
n für 0, 1 oder 2 steht und
B und X die oben angegebene Bedeutung haben.

3. Antimykotisches Mittel gemäß Anspruch 1, gekennzeichnet durch einen Gehalt an 1-(2,4-Dichlorphenoxy)-3,3-dimethyl-4-fluor-1-(imidazol-1-yl)-2-butanon.
4. Antimykotisches Mittel gemäß Anspruch 1, gekennzeichnet durch einen Gehalt an 1-(4-Chlorphenoxy)-3,3-di-(fluormethyl)-1-(imidazol-1-yl)-2-butanon.
5. Antimykotisches Mittel gemäß Anspruch 1, gekennzeichnet durch einen Gehalt an 1-(4-Fluorphenoxy)-3,3-di-(fluormethyl)-1-(imidazol-1-yl)-2-butanon.
6. Antimykotisches Mittel gemäß Anspruch 1, gekennzeichnet durch einen Gehalt an 1-(4-Chlorphenoxy)-3,3-dimethyl-4-fluor-1-(imidazol-1-yl)-2-butanol.
7. Antimykotisches Mittel gemäß Anspruch 1, gekennzeichnet durch einen Gehalt an 1-(4-Bromphenoxy)-3,3-dimethyl-4-fluor-1-(imidazol-1-yl)-2-butanol.
8. Verfahren zur Herstellung eines antimykotischen Mittels gemäß Anspruch 1, dadurch gekennzeichnet, daß man fluorierte 1-Imidazolyl-butan-Derivate gemäß der allgemeinen Formel I in Anspruch 1 mit inerten, nichttoxischen, pharmazeutisch geeigneten Trägerstoffen vermischt.

**Claims**

1. Antimycotic agent, characterised in that it contains at least one fluorinated 1-imidazolyl-butane derivative of the general formula I

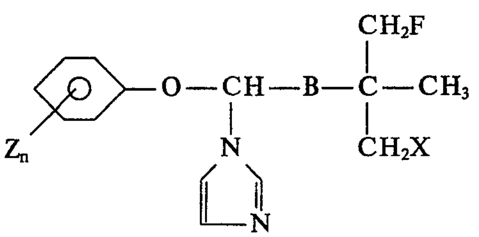

(I)

in which

B represents the keto group or the CH(OH)-grouping,
X represents hydrogen or fluorine,

Z represents halogen, alkyl with 1 to 4 carbon atoms, nitro, cyano, alkoxycarbonyl with 1 to 4 carbon atoms in the alkyl part, or phenyl which is optionally substituted by halogen, and
n represents 0, 1, 2 or 3,

and/or physiologically acceptable acid addition salts and/or metal complexes thereof.

2. Antimycotic agent according to Claim 1, characterised in that it contains at least one fluorinated 1-imidazolyl-butane derivative of the general formula 1 in Claim 1, in which

Z represents halogen, methyl, ethyl, nitro, cyano, methoxycarbonyl, ethoxycarbonyl, phenyl or chlorophenyl,
n represents 0, 1 or 2 and
B and X habe the meaning given above.

3. Antimycotic agent according to Claim 1, characterised in that it contains 1-(2,4-dichlorophenoxy)-3,3-dimethyl-4-fluoro-1-(imidazol-1-yl)-2-butanone.

4. Antimycotic agent according to Claim 1, characterised in that it contains 1-(4-chlorophenoxy)-3,3-di-(fluoromethyl)-1-(imidazol-1-yl)-2-butanone.

5. Antimycotic agent according to Claim 1, characterised in that it contains 1-(4-fluorophenoxy)-3,3-di-(fluoromethyl)-1-(imidazol-1-yl)-2-butanone.

6. Antimycotic agent according to Claim 1, characterised in that it contains 1-(4-chlorophenoxy)-3,3-dimethyl-4-fluoro-1-(imidazol-1-yl)-2-butanol.

7. Antimycotic agent according to Claim 1, characterised in that it contains 1-(4-bromophenoxy)-3,3-dimethyl-4-fluoro-1-(imidazol-1-yl)-2-butanol.

8. Process for the preparation of an antimycotic agent according to Claim 1, characterised in that fluorinated 1-imidazolyl-butane derivatives according to the general formula I in Claim 1 are mixed with inert, non-toxic, pharmaceutically suitable excipients.

**Revendications**

1. Agent antimycotique, caractérisé en ce qu'il contient au moins un dérivé fluoré de 1-imidazolyl-butane de formule générale I:

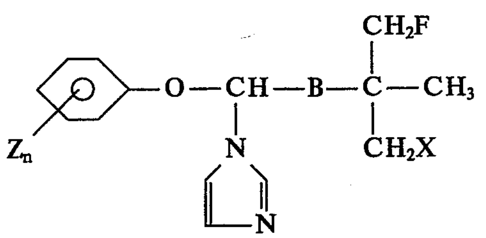

(I)

dans laquelle

B représente le groupe céto ou le groupement CH(OH),
X représente un atome d'hydrogène ou un atome de fluor,
Z représente un atome d'halogène, un groupe alkyle contenant 1 à 4 atomes de carbone, un groupe nitro, un groupe cyano, un groupe alcoxycarbonyle contenant 1 à 4 atomes de carbone dans la fraction alkyle, de même qu'un groupe phényle éventuellement substitué par un atome d'halogène, et
n est égal à 0, 1, 2 ou 3,

et/ou ses complexes métalliques et/ou ses sels d'addition d'acide physiologiquement compatibles.

2. Agent antimycotique suivant la revendication 1, caractérisé en ce qu'il contient au moins un dérivé fluoré de 1-imidazolyl-butane de formule générale I suivant la revendication 1, formule dans laquelle:

Z représente un atome d'halogène, un groupe méthyle, un groupe éthyle, un groupe nitro, un groupe cyano, un groupe méthoxycarbonyle, un groupe éthoxycarbonyle, un groupe phényle ou un groupe chlorophényle,
n est égal à 0, 1 ou 2, et
B et X ont les significations indiquées ci-dessus.

3. Agent antimycotique suivant la revendication 1, caractérisé en ce qu'il contient la 1-(2,4-dichlorophénoxy)-3,3-diméthyl-4-fluoro-1-(imidazol-1-yl)-2-butanone.

4. Agent antimycotique suivant la revendication 1, caractérisé en ce qu'il contient la

1-(4-chlorophénoxy)-3,3-di-(fluorométhyl)-1-(imidazol-1-yl)-2-butanone.

5. Agent antimycotique suivant la revendication 1, caractérisé en ce qu'il contient la 1-(4-fluorophénoxy)-3,3-di-(fluorométhyl)-1-(imidazol-1-yl)-2-butanone.

6. Agent antimycotique suivant la revendication 1, caractérisé en ce qu'il contient le 1-(4-chlorophénoxy)-3,3-diméthyl-4-fluoro-1-(imidazol-1-yl)-2-butanol.

7. Agent antimycotique suivant la revendication 1, caractérisé en ce qu'il contient le 1-(4-bromophénoxy)-3,3-diméthyl-4-fluoro-1-(imidazol-1-yl)-2-butanol.

8. Procédé de préparation d'un agent antimycotique suivant la revendication 1, caractérisé en ce qu'on mélange des dérivés fluorés de 1-imidazolyl-butane répondant à la formule générale I de la revendication 1 avec des substances supports inertes, non toxiques et pharmaceutiquement appropriées.